# EUROPEAN PATENT APPLICATION

(11) **EP 3 181 549 A1**
(43) Date of publication of application: **21.06.2017**
(21) Application number: 15460122.3
(22) Date of filing: 17.12.2015
(51) Int. Cl.: C07D 209/48, A61K 31/4035

(54) **PROCESS FOR THE PREPARATION OF APREMILAST**

(71) Applicant: Zaklady Farmaceutyczne Polpharma SA, 83-200 Starogard Gdanski (PL)
(72) Inventor: Podwysocka, Dominika, 83-000 Pruszcz Gdanski (PL); Majewski, Arkadiusz, 80-462 Gdansk (PL); Szulc, Marcin, 83-200 Starogard Gdanski (PL); Oracz, Monika, 97-360 Kamiensk (PL)
(74) Representative: Obrycki, Pawel Andrzej

(57) **Abstract**

This invention provides a process for preparing apremilast, comprising reacting compounds of formula I and formula II or a salt thereof where neither acetic nor formic acid are present.

## Description

The present invention relates to a process for the preparation of a pharmaceutical agent, and particularly to a process for preparing apremilast.

Arthritis represents a major global health problem, for example, arthritis is the most common cause of disability in the United States of America, affecting 20 million people and placing severe restrictions on their physical capabilities.

The term "arthritis" encompasses a variety of disease states which typically involve inflammation of one or more joints. Patients most typically present with joint pain that is a direct result of inflammation occurring around the joint. The pain is often localised to the affected joint and provides a constant source of physical distress for patients.

Psoriasis is a chronic inflammatory skin disease that can lead to significant physical and psychological distress for patients.

Inflammation is an omnipresent symptomatic feature of both arthritis and psoriasis. Aside from sharing this common symptom the disease states overlap further in that a particular form of arthritis exists which is directly attributable to psoriasis. Psoriatic arthritis is known to develop in approximately 30% of people who already suffer from psoriasis. The disease impacts upon the patients' range of motion, and patients typically present with swelling, stiffness and pain within and around the joints. Physical fatigue is also a common complaint among sufferers. Although the exact cause of the disease has not been elucidated, it has been postulated that the prolonged skin inflammation experienced by psoriasis sufferers can inflict proximal damage to neighbouring joints, which then manifests as arthritis.

Traditionally arthritic conditions (e.g. psoriatic arthritis) have been treated and controlled by administering patients with nonsteroidal anti-inflammatory drugs (NSAIDS) i.e. ibuprofen and naproxen for mild symptomatic pain, and diclofenac and indomethacin in more serious circumstances. Although providing temporary relief to the patient, long-term use of these agents is less than satisfactory owing to adverse effects (including gastrointestinal bleeding, kidney damage and cardiovascular damage) hence limiting their utility. More recent studies have investigated the pathogenesis of psoriasis and psoriatic arthritis at the molecular level. Elucidation of the role of tumour necrosis factor alpha (TNFα) in inflammation pathways has led to the development of selective biologic agents which are able to inhibit TNFα in vivo. Reducing the intracellular activity of TNFα allows for mediation and reduction of inflammation.

Although better suited than traditional therapies, biologics too suffer from drawbacks, namely high cost (to the patient) and the requirement for intravenous administration. For these reasons new oral treatment options have emerged. These therapies operate analogously to the biologic treatment options in targeting inflammation pathways at the molecular level. Amongst the agents that have been developed are phosphodiesterase IV (PDE4) inhibitors. PDE4 is a cell signalling protein that is involved in systemic inflammation pathways, and acts upstream of TNFα. Inhibition of PDE4 ultimately reduces TNFα production, in turn suppressing immune responses and reducing inflammation. Apremilast is one such orally available PDE4 inhibitor used in the treatment of psoriasis and psoriatic arthritis.

Apremilast is named *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide. Apremilast is represented structurally by the formulae below.

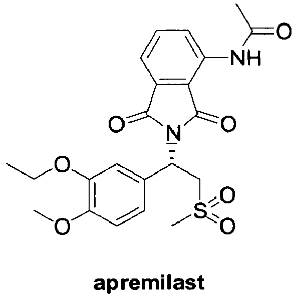

Apremilast is indicated for the treatment of patients presenting with moderate to severe plaque psoriasis and psoriatic arthritis. The drug is commercially marketed by Celgene Ltd under the trade name Otezla® and is formulated as an oral film-coated tablet in a size amount of 10, 20 or 30 mg.

WO 03/080049 details a synthesis of apremilast:

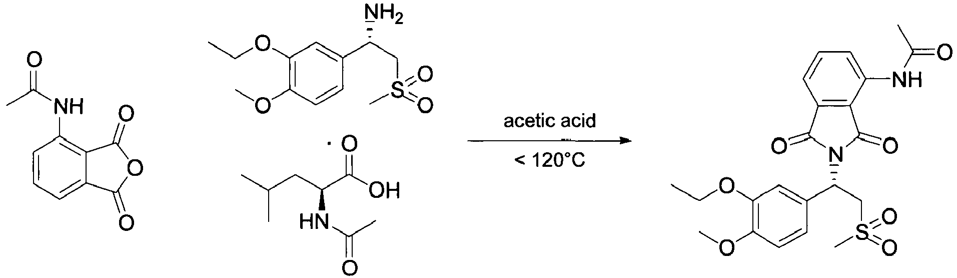

Various other syntheses of apremilast are known in the art, however they often lack efficiency, require prolonged reaction times, require the use of onerous work-up techniques and lead to the generation of undesired side products.

The synthetic methodology employed to produce multi-kilogram quantities of a market-approved pharmaceutically active ingredient, for example apremilast, differ greatly from the methodologies employed at the discovery stage. Early stage drug discovery campaigns typically require the production of 5-10 mg of material (per molecule of interest). Such an amount allows for thorough characterisation of physical and chemical parameters, and moreover represents a workable quantity for use in biological assessment. Synthetic chemists operating at the discovery level generally do not assess whether a synthetic route taken to access a molecule of interest, is indeed scalable. In fact, it would be prima facie difficult to predict accurately whether a step conducted at small-scale would in fact be amenable to being implemented upon a large scale.

Large-scale chemical synthesis (or process chemistry) concerns the development, optimisation and production of multi-kilogram and multi-tonne quantities of pharmaceutically active ingredients and speciality bulk chemicals. Consequently, and owing to economies of scale, any small improvement in a given reaction parameter is particularly economically significant. Therefore, optimisation of a large-scale synthesis that provides, for example, an increase in chemical yield, decrease in reaction time, decrease in reaction temperature, decrease in amount of catalyst or solvent used, increase in the favourability of reagents, reduction in side-product formation, a more environmentally benign synthesis or increase in chemical purity is of interest both to chemical manufacturers and suppliers. Moreover, step optimisation that reduces the need for multiple or hard-to-perform purifications are particularly beneficial. Conventional column chromatography and high-performance liquid chromatography (HPLC) are ubiquitous techniques, and resemble the cornerstone of the discovery chemist's purification arsenal. Unfortunately, these techniques are unsuitable for the production of multi-kilogram and multi-tonne quantities, yet begrudgingly, such purifications will have to be performed if the target is of high value and no feasible alternative exists. Consequently, any improvement in the ease of purification or isolation, through telescoping (wherein two or more, previously independent synthetic transformations converge into a "single" process and therefore require only one purification step) of synthetic procedures, or the identification of an intermediate for recrystallisation, or precipitation, or removal of impurities through conversion into a transient intermediate, or substitution for a cheaper, more environmentally friendly or less toxic reagent, provide an attractive and economically desirable goal. Practical achievement of this goal is however not straightforward, and even careful optimisation of each individual parameter of a synthetic step (on a small- or large-scale) will often fail to provide a workable advantage within a large-scale synthesis.

For pharmaceuticals currently in development and those presently marketed, there remains a need in the art for improving both the overall synthesis of such entities and the individual constituent transformations (e.g. step-to-step efficacy and increasing product purity) within the syntheses.

Accordingly the present invention provides a process for preparing apremilast, 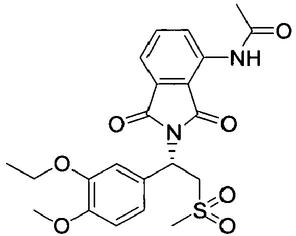 comprising reacting compounds of formula I and formula II or a salt thereof 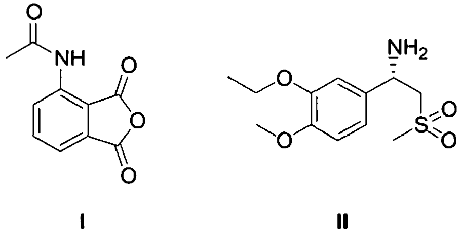 where neither acetic nor formic acid are present.

The present invention entails a condensation reaction between an amino-substituted phthalic anhydride derivative and a primary amine.

In accordance with the present invention, there is provided a process for the preparation of apremilast comprising reacting compounds of formula I and formula II or a salt thereof, where the reaction is carried out in the absence of a protic acid.

The term protic acid should be understood as encompassing acids that can be described by both, or either of the Arrhenius acid and Brønsted-Lowry acid definitions.

An Arrhenius acid is a substance that, when added to water, increases the concentration of protons (H⁺) ions in the water. For the avoidance of doubt, the increase of protons in water will in fact be represented by an increase in the hydronium ion H₃O⁺ as protons per se cannot subsist in water. A Brøonsted-Lowry acid is a species that donates a proton to a Brønsted-Lowry base (a proton acceptor). The following equations demonstrate the use and applicability of each of the Arrhenius and Brønsted-Lowry definitions, the protic acid exemplified being acetic acid:

(a) CH₃COOH + H₂O ⇄ CH₃COO⁻ + H₃O⁺

(b) CH₃COOH ⁺ NH₃ ⇄ CH₃COO⁻ + NH₄⁺

Equation (a) can be described by both definitions of protic acid. First, acetic acid can be said to act as an Arrhenius acid, because following addition to, or reaction with water, the concentration of H₃O⁺ increases. Secondly, acetic acid is also acting as a Bronsted-Lowry acid by donating a proton to water. Equation (b) highlights a similar situation wherein acetic acid donates a proton to ammonia and thus again behaves like a Brønsted-Lowry acid, however equation (b) cannot be described by the Arrhenius definition, because the reaction is occurring in the absence of water.

Furthermore, and in accordance with the present invention, it should be understood that the term protic acid is intended to cover protic acids that when added to water in an amount sufficient to form a 1 molar aqueous solution at 25°C, the resulting solution will have a pH less than 7 when measured by conventional methods e.g. a pH meter.

Examples of typical protic acids that are considered to fall within the definitions as set out above are acetic acid, formic acid, sulfuric acid, hydrochloric acid, p-toluenesulfonic acid, methanesulfonic acid, trifluoromethanesulfonic acid, trifluoroacetic acid, phosphoric acid, benzoic acid, citric acid, tartaric acid, nitric acid, hydrobromic acid, hydrofluoric acid, carbonic acid, potassium bisulfate, anthranilic acid, ethylenediaminetetraacetic acid, fumaric acid, glycolic acid, lactic acid, maleic acid, malonic acid, oxalic acid, peracetic acid, propionic acid, phthalic acid, salicyclic acid, succinic acid and sulfamic acid.

It is preferred that the present invention is performed in the absence of protic acid, for example in the absence of typical protic acids listed above. It should also be noted that the list is not exhaustive and is not in any way intended to be limiting upon the scope of the invention.

The term "acidic proton" is a term of the art and is used to describe protons which have the ability to act as acids in a given chemical environment. Generally, and in accordance with the present invention, "acidic protons" should be understood as being inherently more acidic in comparison to alkyl protons i.e. protons directly bonded to a carbon atom in an sp3, sp2 or sp hybridisation state.

Protons that are generally considered "acidic protons" are typically directly bonded to an oxygen or nitrogen atom. For example, a molecule containing one or more protons in such an environment e.g. an amide proton could lead to an interpretation that the molecule per se is a protic acid. According to the present invention, where there is provided a process for the preparation of apremilast comprising reacting compounds of formula I and formula II or a salt thereof in the absence of a protic acid, it should be understood that molecules possessing protons in the aforementioned environments, that is, environments in which a proton is considered an "acidic proton", are not considered to be protic acids.

Apremilast per se and the compound of formula I contain protons that can be considered inherently acidic in accordance with the definitions recited hereinabove. However it is submitted that these protons are not considered protic acids in accordance with the present invention.

Alternatively, if the acidic protons contained within apremilast and the compound of formula I do lead to the consideration of the product (apremilast) and reagent (a compound of formula I) as protic acids, then in accordance with the present invention, there is provided a process for preparing apremilast, 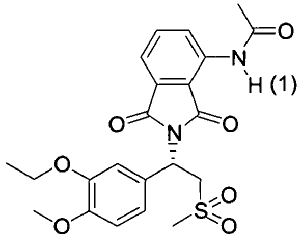 comprising reacting compounds of formula I and formula II or a salt thereof 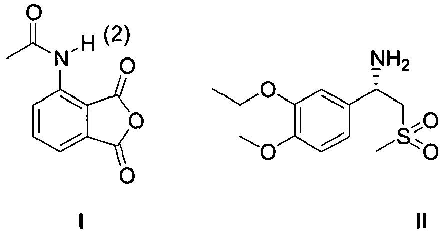 wherein the only protic acid present is generated from protons (1) and (2).

In accordance the present invention, the process for preparing apremilast is preferabably conducted in a solvent (i.e. an organic solvent) or a mixture of solvents.

It is known in the art which particular solvent is likely to be most appropriate and most effective for each category of chemical transformation. For example, water- or air-sensitive transformations require the use of non-aqueous, non-protic solvents such as acetonitrile, tetrahydrofuran, dioxane, dimethylsulfoxide dichloromethane, chloroform or *N*-methylpyrrolidine. Furthermore, water- or air-sensitive reactions may also require an atmosphere of inert gas (to prevent atmospheric water from compromising a transformation) typical inert gases include nitrogen and argon. Alternatively, reactions requiring the use of an aqueous reagent (e.g. aqueous hydrochloric acid or aqueous sodium hydroxide) may require the use of one or more organic co-solvents. In this context, an organic co-solvent is added to aid the dissolution, or partial dissolution of a less polar organic reagent. Typical organic co-solvents for use with such aqueous reagents include, toluene, alcohols (methanol, ethanol, propanol), tetrahydrofuran and dimethylsulfoxide. The only limitation in regard to reaction efficacy when using organic co-solvents with aqueous solvents is the requirement for some degree of miscibility of the organic co-solvent with water. To aid problems with miscibility and or reaction efficacy a phase transfer catalyst is employed (e.g. tetrabutylammonium bromide).

Solvents are traditionally categorised into two categories, polar and non-polar. More specifically solvents are allocated into each category according to their dielectric constant. It is generally accepted that solvents displaying a dielectric constant of less than 15 are considered to be non-polar solvents, and solvents displaying a dielectric constant of greater than or equal to 15 are considered to be polar solvents.

Preferred solvents for use in preparing apremilast according to the present invention are polar solvents. Particularly preferred solvents for use in preparing apremilast are alkylacetamide-containing solvents.

Alkylacetamides are solvents that comprise carbon, hydrogen, oxygen and nitrogen only, and whereas the term acetamide represents solvents wherein the amine (-NH₂) group is unsubsituted, alkylacetamide represents solvents wherein the amine group is mono- (-NR₁H) or di- (NR₁R₂) substituted with one or more alkyl groups, wherein the alkyl groups (R₁) and (R₂) can be identical or different. The alkyl groups present upon the solvent used in conjunction with the present invention are C₁-C₁₀ alkyl groups.

Typical examples of alkylacetamide solvents that can be used in conjunction with the present invention are, *N,N*-dimethylacetamide, *N,N*-dimethylformamide, *N*-methylacetamide, *N-*methylformamide, *N*-methyl-2-pyrrolidone and pyrrolidone. It is particularly preferred wherein the solvent used in conjunction with the present invention is *N,N*-dimethylacetamide or *N,N-*dimethylformamide. It is most preferred wherein the solvent used in conjunction with the present invention is *N,N*-dimethylacetamide.

In accordance with the present invention, it is preferred that the process for preparing apremilast, comprising reacting compounds of formula I and formula II or a salt thereof where neither acetic nor formic acid are present, is conducted in an alkylacetamide solvent.

It is also preferred that the process for preparing apremilast, comprising reacting compounds of formula I and formula II or a salt thereof in the absence of a protic acid, is conducted in an alkylacetamide solvent.

It is also preferred that the process for preparing apremilast, comprising reacting compounds of formula I and formula II or a salt thereof where neither acetic nor formic acid are present, is conducted in a *N,N*-dimethylacetamide or *N,N*-dimethylformamide solvent. It is particularly preferred when the solvent is *N,N*-dimethylacetamide.

It is also preferred that the process for preparing apremilast, comprising reacting compounds of formula I and formula II or a salt thereof in the absence of a protic acid, is conducted in a *N,N-*dimethylacetamide or *N,N*-dimethylformamide solvent. It is particularly preferred when the solvent is *N,N*-dimethylacetamide.

The process for preparing apremilast is advantageously conducted at a temperature obtainable by the application of simple and non-expensive heating technologies. Preferably the process of the present invention is conducted at a temperature of from 90°C to 150°C.

The present invention requires non-expensive heating technologies in order to obtain the most preferred reaction temperature of from 110°C to 120°C. Advantageously, the process for preparing apremilast requires a relatively short reaction time.

It is preferred where the process for preparing apremilast, comprising reacting compounds of formula I and formula II or a salt thereof, where neither acetic nor formic acid are present and wherein the solvent is an alkylacetamide solvent, that the reaction is conducted at a temperature of from 110°C to 120°C and the duration of the reaction is 1 to 5 hours. It is more preferred wherein the duration of the reaction is 3 hours. It is also preferred where the process for preparing apremilast, comprising reacting compounds of formula I and formula II or a salt thereof, in the absence of a protic acid and wherein the solvent is an alkylacetamide solvent, that the reaction is conducted at a temperature of from 110°C to 120°C and the duration of the reaction is 1 to 5 hours. It is more preferred wherein the duration of the reaction is about 3 hours, or is 3 hours.

It is also preferred where the process for preparing apremilast, comprising reacting compounds of formula I and formula II or a salt thereof, where neither acetic nor formic acid are present and wherein the solvent is *N,N*-dimethylacetamide or *N,N*-dimethylformamide, that the reaction is conducted at a temperature of from 110°C to 120°C and the duration of the reaction is 1 to 5 hours. It is more preferred where the duration of the reaction is 3 hours and wherein the solvent is *N,N-*dimethylacetamide. It is also preferred where the process for preparing apremilast, comprising reacting compounds of formula I and formula II or a salt thereof, in the absence of a protic acid and wherein the solvent is *N,N*-dimethylacetamide or *N,N*-dimethylformamide, that the reaction is conducted at a temperature of from 110°C to 120°C and the duration of the reaction is 1 to 5 hours. It is more preferred where the duration of the reaction is 3 hours and wherein the solvent is *N,N-*dimethylacetamide.

The term a salt thereof used in conjunction with a compound of formula II means any pharmaceutically acceptable salt thereof, and for example, typically refers to and includes at least the N-acetyl-L-leucine salt of a compound of formula II.

The N-acetyl-L-leucine salt of a compound of formula II is represented structurally below.

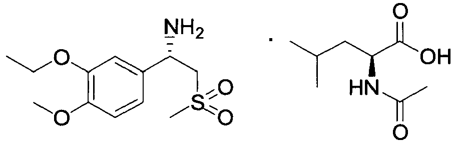

In accordance with the present invention, there is provided a process for the preparation of apremilast comprising reacting compounds of formula I and formula II or a salt thereof in the absence of a protic acid. Where it is preferred that a salt of formula II is used, it is most preferred that the salt of formula II is the N-acetyl-L-leucine salt. In such circumstances the N-acetyl-L-leucine salt is not to be understood as a protic acid.

Alternatively, if the acidic protons contained within apremilast, the compound of formula I and the N-acetyl-L-leucine salt of a compound of formula II lead to the consideration of the product (apremilast) and reagents (a compound of formula I and the N-acetyl-L-leucine salt of a compound of formula II) as protic acids, then in accordance with the present invention, there is provided a process for preparing apremilast, 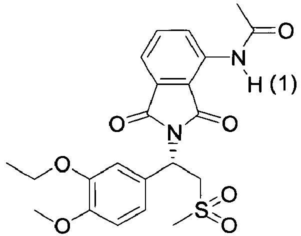 comprising reacting compounds of formula I and formula II or a salt thereof 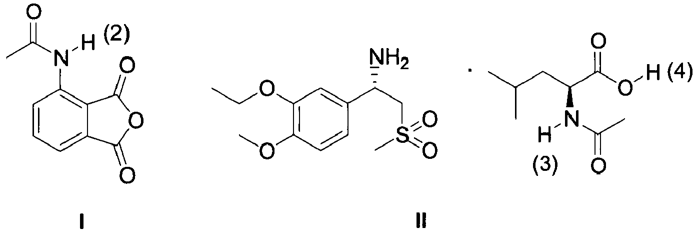 wherein the only protic acid present is generated from protons (1), (2), (3) and (4).

It is preferred where the process for preparing apremilast, comprising reacting compounds of formula I and formula II or a salt thereof, wherein the only protic acid present is generated from protons (1), (2), (3) and (4) and wherein the solvent is an alkylacetamide solvent, that the reaction is conducted at a temperature of from 110°C to 120°C and the duration of the reaction is 1 to 5 hours. It is more preferred wherein the duration of the reaction is 3 hours.

It is also preferred where the process for preparing apremilast, comprising reacting compounds of formula I and formula II or a salt thereof, wherein the only protic acid present is generated from protons (1), (2), (3) and (4) and wherein the solvent is *N,N*-dimethylacetamide or *N,N-*dimethylformamide, that the reaction is conducted at a temperature of from 110°C to 120°C and the duration of the reaction is 1 to 5 hours. It is more preferred where the duration of the reaction is 3 hours and wherein the solvent is *N,N*-dimethylacetamide.

The process of the present invention is performed in one pot. The term "one pot" should be understood as referring to a single operational step, i.e. where reagents are added consecutively into one pot, and no intermediate is isolated.

The process of the present invention provides a one pot synthesis, minimising both the requirement for multiple purification steps and for example the amount of solvent needed and used in multistep syntheses. The process also reduces the operational complexity associated with chemical reactions conducted using multiple steps in multiple different reaction vessels.

Purification of apremilast is preferably simplified following the removal of the reaction solvent by, for example, reduced pressure evaporation or distillation.

Purification of apremilast can be performed using methods known in the art. Compounds can be purified by, for example, normal phase liquid chromatography, reversed phase and normal phase high-performance liquid chromatography and recrystallisation or salt formation. Furthermore, the purity of apremilast can be analysed and measured using high-performance liquid chromatography or chiral high-performance liquid chromatography. Preferred solvents for HPLC analysis and purification are H₂O/MeCN (water/acetonitrile). Typically, HPLC analysis can be performed using a 100 x 2.1 mm Waters Acquity C18 BEH column, where detection is set at 230nm, and wherein elution is conducted using a H₂O/MeCN gradient (97/3 to 20/80) over a period of 15 minutes.

It is also provided that following the process of the present invention, the apremilast obtained is crystallised from a mixture of acetone and ethanol. The apremilast obtained is crystallised from a mixture of acetone and ethanol to afford pure apremilast. It is preferred that apremilast is dissolved in a mixture of acetone and ethanol, at a temperature at which the mixture is boiling. Upon dissolution, the mixture is allowed to cool to ambient temperature (about 25°C or room temperature) and stirred for 3 to 6 hours. It is also preferred that apremilast is dissolved in a solution of boiling acetone, and following dissolution a solution of boiling ethanol is added thereto, to form a mixture of boiling acetone and ethanol. Upon addition of the boiling ethanol the mixture is allowed to cool to ambient temperature (about 25°C or room temperature) and stirred for 3 to 6 hours.

This invention provides a process for preparing apremilast, comprising reacting compounds of formula I and formula II or a salt thereof where neither acetic nor formic acid are present.

Accordingly, it can be seen that the present invention provides a one-pot process for providing apremilast, the process comprises a condensation reaction between an amino-substituted phthalic anhydride derivative and a primary amine where neither acetic nor formic acid are present.

The present invention will now be described with reference to the examples, which are not intended to be limiting.

### Examples

### Example 1

### N-[2-[(1S)-1-(3-ethoxy-4-methoxyphenyl)-2-(methylsulfonyl)ethyl]-1,3-dioxo-2,3-dihydro-1H-isoindol-4-yl]acetamide

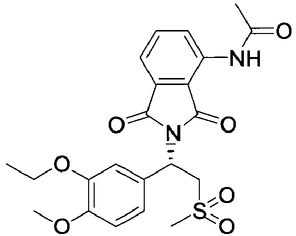

*N*-(1,3-dioxo-1,3-dihydro-2-benzofuran-4-yl)acetamide (9.65 g) was dissolved in dimethylacetamide (75 mL) at ambient temperature (20-25°C). (*S*)-1-(3-ethoxy-4-methoxyphenyl)-2-(methylsulfonyl)ethylamine *N*-acetyl-L-leucine salt (20 g) was suspended in dimethylacetamide (75 mL) and the suspension was added to the solution of *N*-(1,3-dioxo-1,3-dihydro-2-benzofuran-4-yl)acetamide in dimethylacetamide. The resulting mixture was heated to 110-120°C under flow of inert gas and the reaction mixture was stirred for 2-4 hours. Upon completion of the reaction (i.e. 2-4 hours at 110-120°C) the solution was cooled to ambient temperature. Ethyl acetate (400 mL) and 2% aqueous solution of Na₂CO₃ (400 mL) were added to the reaction mixture and it was allowed to stir for 10 min at 20-30°C. After phase separation the ethyl acetate was separated and the aqueous phase was extracted with ethyl acetate (1 x 133 mL). The combined ethyl acetate phases were washed sequentially with brine (3 x 133 mL) and water (1 x 133 mL). The combined ethyl acetate was then allowed to stir with charcoal (2 g) for 30 minutes. The charcoal was removed via filtration and the ethyl acetate was evaporated under reduced pressure (200-30mbar) at 50-65°C giving *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide as a crude residue.

### Example 2

### Crystallisation (i) of N-[2-[(1S)-1-(3-ethoxy-4-methoxyphenyl)-2-(methylsulfonyl)ethyl]-1,3-dioxo-2,3-dihydro-1H-isoindol-4-yl]acetamide

The residue obtained in Example 1 (10 g) was dissolved in a mixture of acetone (30 mL) and ethanol (60 mL) at boiling temperature and charcoal (1 g) was added. The mixture was allowed to stir for 30 minutes at boiling temperature. The charcoal was filtered off and washed with a mixture of acetone (5mL) and ethanol (10 mL). The mixture was allowed to cool to a designated temperature (A, B, C, D - see table below) and stirred for 3-6 hours. The solid obtained in each case was filtered under reduced pressure and the filter cake was washed with ethanol (2 x 20 mL). Drying the solid under reduced pressure at 60°C gave *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide with a purity above 99.50% and (80-90% yield) (XRPD: form B).

### Crystallisation (ii) of N-[2-[(1S)-1-(3-ethoxy-4-methoxyphenyl)-2-(methylsulfonyl)ethyl]-1,3-dioxo-2,3-dihydro-1H-isoindol-4-yl]acetamide

The residue obtained in Example 1 was dissolved in acetone (30 mL) at boiling temperature and charcoal (1 g) was added. The mixture was allowed to stir for 30 minutes at boiling temperature. The charcoal was filtered off and washed with acetone (5 mL). A solution of boiling ethanol (70 mL) was then added to the solution. The mixture was allowed to cool to a designated temperature (A, B, C, D - see table below) and stirred for 3-6 hours. The solid obtained was filtered under reduced pressure and the filter cake was washed with ethanol (2 x 20 mL). Drying the solid under reduced pressure at 60°C gave *N*-[2-[(1*S*)-1-(3-ethoxy-4-methoxyphenyl)-2-(methylsulfonyl)ethyl]-1,3-dioxo-2,3-dihydro-1 H-isoindol-4-yl]acetamide with a purity above 99.50% and (80-90% yield) (XRPD: form B).

| | Temperature [°C] | Yield [%] | Purity on HPLC [%] |
|---|---|---|---|
| A | 15 - 25 | 82 | >99.50 |
| B | 5 - 15 | 84 | >99.50 |
| C | -5 - -5 | 88 | >99.50 |
| D | -15 - -5 | 90 | >99.50 |

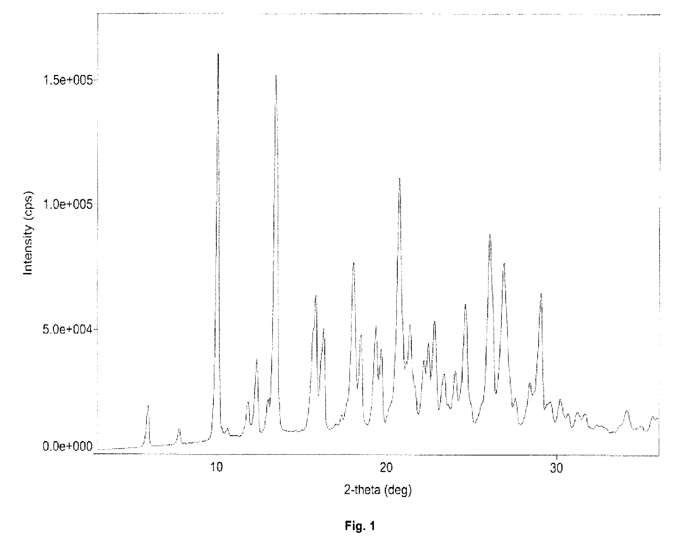

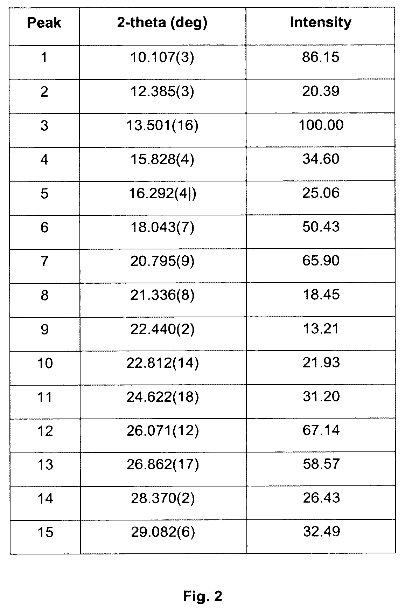

The XRPD spectrum for apremilast polymorphic form B and accompanying peak data is shown in Fig. 1 and Fig 2 respectively.

### Example 3

### N-[2-[(1S)-1-(3-ethoxy-4-methoxyphenyl)-2-(methylsulfonyl)ethyl]-1,3-dioxo-2,3-dihydro-1H-isoindol-4-yl]acetam ide

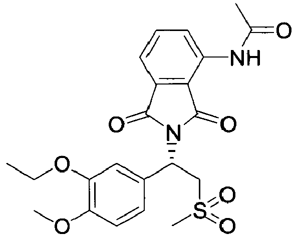

*N*-(1,3-dioxo-1,3-dihydro-2-benzofuran-4-yl)acetamide (215 g, 1.05 mol) was dissolved in dimethylacetamide (1070 mL) at ambient temperature (20-25°C). (*S*)-1-(3-ethoxy-4-methoxyphenyl)-2-(methylsulfonyl)ethylamine *N*-acetyl-L-leucine salt (446 g, 1.00 mol) was suspended in dimethylacetamide (1070 mL) and the suspension was added to the solution of *N*-(1,3-dioxo-1,3-dihydro-2-benzofuran-4-yl)acetamide in dimethylacetamide. The resulting mixture was heated to 110-120°C under flow of inert gas and the reaction mixture was stirred for 3 hours. Upon completion of the reaction (i.e. 3 hours at 110-120°C) the solution was cooled to ambient temperature. Ethyl acetate (8960 mL) and a 2% aqueous solution of Na₂CO₃ (8920 mL) were added to the reaction mixture and allowed to stir for 10 min at 20-30°C. After phase separation the ethyl acetate was separated and the aqueous phase was extracted with ethyl acetate (1 x 1500 mL). The combined ethyl acetate phases were washed sequentially with brine (3 x 1485 mL) and water (3 x 500 mL). The combined ethyl acetate was evaporated under reduced pressure (200-30mbar) at 50-65°C giving *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide as a residue. The HPLC profile of the residue identified apremilast (99.75%) and impurities (0.10%).

The obtained residue was crystallised according to methods as recited in Example 2. Following crystallisation *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide was obtained with a purity above 99.9% and (75% yield, 345 g).

### Comparative Example

### N-[2-[(1S)-1-(3-ethoxy-4-methoxyphenyl)-2-(methylsulfonyl)ethyl]-1,3-dioxo-2,3-dihydro-1H-isoindol-4-yl]acetamide

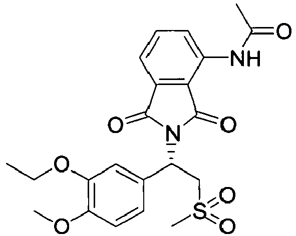

*N*-(1,3-dioxo-1,3-dihydro-2-benzofuran-4-yl)acetamide (215 g, 1.05 mol) was dissolved in acetic acid (2230 mL) at ambient temperature (20-25°C). (*S*)-1-(3-ethoxy-4-methoxyphenyl)-2-(methylsulfonyl)ethylamine *N*-acetyl-L-leucine salt (446 g, 1.00 mol) was suspended in acetic acid (2230 mL) and the suspension was added to the solution of *N*-(1,3-dioxo-1,3-dihydro-2-benzofuran-4-yl)acetamide in dimethylacetamide. The resulting mixture was heated to 118-120°C under flow of inert gas and the reaction mixture was stirred for 16 hours. Upon completion of the reaction (i.e. 16 hours at 110-120°C) the solution was cooled to ambient temperature. Ethyl acetate (8960 mL) was added to the reaction mixture and it was allowed to stir for 10 min at 20-30°C. After phase separation the ethyl acetate was separated and the aqueous phase was extracted with ethyl acetate (1 x 1500 mL). The combined ethyl acetate phases were washed sequentially with brine (3 x 1485 mL), 8% aqueous solution of NaHCO₃ (8920 mL), (water (3 x 2960 mL) and a 2% aqueous solution of Na₂SO₄ (1 x 1000 mL). The combined ethyl acetate was evaporated under reduced pressure (200-30mbar) at 50-65°C giving *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide as a residue. The HPLC profile of the residue identified apremilast (97.21%) and impurities (2.04%).

The obtained residue was crystallised according to methods as recited in Example 2. Following crystallisation *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide was obtained with a purity of 99.5% and (73% yield, 336 g).

In an attempt to increase the purity of the apremilast (above 99.9%) the same crystallisation procedure was repeated, in which *N*-[2-[(1*S*)-1-(3-ethoxy-4-methoxyphenyl)-2-(methylsulfonyl)ethyl]-1,3-dioxo-2,3-dihydro-1H-isoindol-4-yl]acetamide was obtained with a purity of 99.85% and (90% yield - based upon the material obtained from the first crystallisation, 302 g) giving a total yield of apremilast (across both crystallisations) of 65%.

### Example 4

### N-[2-[(1S)-1-(3-ethoxy-4-methoxyphenyl)-2-(methylsulfonyl)ethyl]-1,3-dioxo-2,3-dihydro-1H-isoindol-4-yl]acetamide

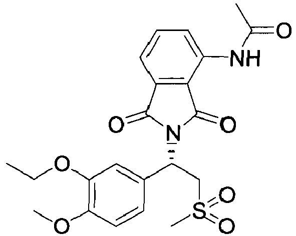

*N*-(1,3-dioxo-1,3-dihydro-2-benzofuran-4-yl)acetamide (33.86 g) was dissolved in dimethylformamide (345 mL) at ambient temperature (20-25°C). (*S*)-1-(3-ethoxy-4-methoxyphenyl)-2-(methylsulfonyl)ethylamine *N*-acetyl-L-leucine salt (70 g) was suspended in dimethylformamide (280 mL) and the suspension was added to the solution of *N*-(1,3-dioxo-1,3-dihydro-2-benzofuran-4-yl)acetamide in dimethylacetamide. The resulting mixture was heated to 110-120°C under flow of inert gas and the reaction mixture was stirred for 3 hours. Upon completion of the reaction (i.e. 3 hours at 110-120°C) the solution was cooled to ambient temperature. Ethyl acetate (1400 mL) and 2% aqueous solution of Na₂CO₃ (1400 mL) were added to the reaction mixture and it was allowed to stir for 10 min at 20-30°C. After phase separation the ethyl acetate was separated and the aqueous phase was extracted with ethyl acetate (1 x 467 mL). The combined ethyl acetate phases were washed sequentially with brine (3 x 467 mL) and water (1 x 467 mL). The combined ethyl acetate was evaporated under reduced pressure (200-30mbar) at 65°C giving *N*-[2-[(1S)-1-(3-ethoxy-4-methoxyphenyl)-2-(methylsulfonyl)ethyl]-1,3-dioxo-2,3-dihydro-1H-isoindol-4-yl]acetamide as a crude residue.

The crude residue was then dissolved in acetone (210 mL) at boiling temperature. A solution of boiling ethanol (840 mL) was then added to the solution. The mixture was allowed to cool gradually over 5 hours to 0-5°C and then allowed to stir for 12 hours.

The solid obtained was filtered under reduced pressure and the filter cake was washed with ethanol (2 x 105 mL). Drying the solid under reduced pressure at 60°C gave *N*-[2-[(1S)-1-(3-ethoxy-4-methoxyphenyl)-2-(methylsulfonyl)ethyl]-1,3-dioxo-2,3-dihydro-1H-isoindol-4-yl]acetamide with a purity of 99.50% and (83% yield - 60.3 g) (XRPD: form B).

## Claims

1. A process for preparing apremilast, 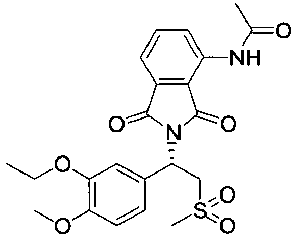 comprising reacting compounds of formula I and formula II or a salt thereof 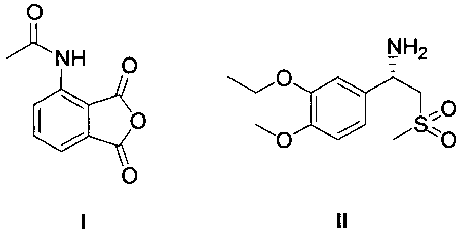 where neither acetic acid nor formic acid are present.

2. A process as claimed in claim 1, where the reaction is carried out in the absence of a protic acid.

3. A process as claimed in claims 1 or 2, where the reaction is carried out in a solvent.

4. A process as claimed in claim 3, wherein the solvent is an alkylacetamide-containing solvent.

5. A process as claimed in claim 4, wherein the alkylacetamide-containing solvent is *N,N-*dimethylacetamide or *N,N*-dimethylformamide.

6. A process as claimed in any one of claims 1 to 5, wherein the reaction is conducted at a temperature of 90 to 150°C.

7. A process as claimed in any one of claims 1 to 6, wherein the duration of the reaction is 1 to 5 hours.

8. A process as claimed in any one of claims 1 to 7, wherein the duration of the reaction is about 3 hours.

9. A process as claimed in any one of claims 1 to 8, wherein the compound of formula II is the N-acetyl-L-leucine salt.

10. A process for crystallising apremilast comprising preparing apremilast as claimed in any of the preceding claims, followed by crystallising apremilast from a mixture of acetone and ethanol.
